# EUROPEAN PATENT APPLICATION

(11) **EP 4 050 338 A1**
(43) Date of publication of application: **31.08.2022**
(21) Application number: 22158948.4
(22) Date of filing: 25.02.2022
(51) Int. Cl.: G01N 33/543, G01N 33/53

(54) **LATEX AGGLUTINATION REACTION SENSITIZER, LATEX AGGLUTINATION REAGENT, AND METHOD FOR MEASURING TARGET SUBSTANCE IN SPECIMEN BY LATEX AGGLUTINATION METHOD USING THE SAME**

(30) Priority: 26.02.2021 JP 2021031241; 26.01.2022 JP 2022010495
(71) Applicant: CANON KABUSHIKI KAISHA, OHTA-KU Tokyo 146-8501 (JP)
(72) Inventor: YAMAUCHI, Fumio, Tokyo (JP); KANAZAKI, Kengo, Tokyo (JP); NATORI, Ryo, Tokyo (JP); KITA, Shoichi, Tochigi (JP)
(74) Representative: WESER & Kollegen Patentanwälte PartmbB

(57) **Abstract**

Provided are a latex agglutination reagent containing polyoxazoline that is preferable for use in a latex agglutination reaction and has a high sensitization action of sensitivity while a non-specific reaction can be suppressed, and a method for measuring a target substance in a specimen by a latex agglutination method.

A latex agglutination reagent used in a method for measuring a target substance in a specimen by a latex agglutination method using an insoluble carrier that carries an antibody or an antigen contains polyoxazoline having a repeating unit represented by the following General Formula 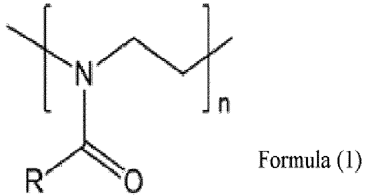 (in Formula (1), R represents a hydrogen atom or a methyl group, an ethyl group, or a propyl group, and n is an integer of 100 or greater).

## Description

### BACKGROUND OF THE INVENTION

### Field of the Invention

The present disclosure relates to a latex agglutination reaction sensitizer, a latex agglutination reagent, and a method for measuring a target substance in a specimen by a latex agglutination method using the same.

### Description of the Related Art

In order to detect and measure specific antigens or antibodies (hereinafter, referred to as a "target substance"), an immunoassay method carried out with an antigen-antibody reaction is used in clinical examinations and the like. Even out of those, the immunoassay method using an insoluble carrier that carries an antibody or an antigen (for example, latex particles) is known as a latex agglutination method.

In an example of the latex agglutination method, a specimen (serum or the like) that may contain a target substance reacts with a latex agglutination reagent that contains particles (also referred to as "antibody sensitized particles") carrying antibodies against the target substance to produce agglomerates by this antigen-antibody reaction, and this agglutination of the agglomerates is visually or optically detected, thereby the target substance capable of being qualitatively and quantitatively measured.

In another example of the latex agglutination method, a specimen that may contain a target substance is mixed with a first reagent (referred to as "R1" in some cases) of the latex agglutination reagent. Next, a sensitized particle dispersion (a second reagent of the latex agglutination reagent, may be referred to as "R2") that contains sensitized particles on which antibodies or antigens against the target substance are immobilized is further mixed. In a case where the target substance is present when the first reagent and the second reagent are mixed, the sensitized particles agglomerate as a result of the antigen-antibody reaction. Therefore, the target substance can be qualitatively and quantitatively measured by visual or optical detection of this agglutination.

In clinical examinations, although a highly sensitive chemiluminescence immunoassay method is also used, the examination time tends to be long since the BF separation (a step of removing unreacted components) is required. By contrast, in the latex agglutination method, although the examination time is short and the examination processing capacity (throughput) is high, the detection sensitivity may be insufficient as compared with the chemiluminescence immunoassay method, and further improvement in the detection sensitivity is demanded.

A polymer compound is used as a sensitizer for the purpose of increasing the sensitivity of the latex agglutination method. More specifically, a latex agglutination reagent containing a sensitizer is used. As a sensitizer, a water-soluble polymer such as polyethylene glycol (PEG) or sodium alginate is added to a reaction system for the purpose of promoting an agglutination reaction. In Japanese Patent Application Laid-Open No. S58-47256 and Japanese Patent No. 2682697, polyethylene glycol that is a nonionic polymer is proposed as a sensitizer. In Japanese Patent Application Laid-Open No. 2003-294753, alginate that is an anionic polymer is proposed as a sensitizer.

By contrast, while these polymers have a sensitization action, the viscosity of a solution may increase or the polymer may be salted out, and a reaction unrelated to the antigen-antibody reaction, that is, a non-specific agglutination reaction may be promoted at the same time. As a result, problems such as an increase in a blank value, a cause of false positives, and a decrease in measurement accuracy may occur. Therefore, there is a demand for a latex agglutination reagent containing a sensitizer exhibiting a sensitization action, suppressing a non-specific reaction, and having good measurement accuracy.

In Japanese Patent No. 3632856, polyoxazoline that is a nonionic polymer is proposed as an additive for enhancing chemiluminescence detection.

By contrast, the sensitization effect of polyoxazoline in a method for measuring a target substance in a specimen by a latex agglutination method using an insoluble carrier that carries an antibody or an antigen has not been known so far.

### SUMMARY OF THE INVENTION

Therefore, an object of the present disclosure is to provide a latex agglutination reagent containing a latex agglutination reaction sensitizer that is preferable for use in a latex agglutination method using an insoluble carrier that carries an antibody or an antigen, exhibits a sensitization action while a non-specific reaction is suppressed, has good measurement accuracy, and does not exist before, and a method for measuring a target substance in a specimen by a latex agglutination method using the same.

As a result of diligent studies to solve the above problems, the present inventors have achieved the above described object by using a latex agglutination reagent containing polyoxazoline as the latex agglutination reaction sensitizer.

That is, the present disclosure relates to a latex agglutination reagent used in the method for measuring a target substance in a specimen by a latex agglutination method using an insoluble carrier that carries an antibody or an antigen contains polyoxazoline (latex agglutination reaction sensitizer) having a repeating unit represented by the following General Formula (1) 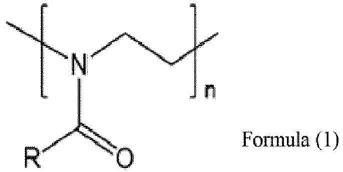 (in Formula (1), R represents a hydrogen atom or a methyl group, an ethyl group, or a propyl group, and n is an integer of 100 or greater).

The present disclosure also relates to a latex agglutination reagent kit including the above described latex agglutination reagent as a constituent component. The present disclosure further relates to a method for measuring a target substance in a specimen by a latex agglutination method using an insoluble carrier that carries an antibody or an antigen, in which the above described latex agglutination reagent or the latex agglutination reagent kit is used in the method for measuring a target substance in a specimen.

Further features of the present disclosure will become apparent from the following description of exemplary embodiments.

### DESCRIPTION OF THE EMBODIMENTS

Hereinafter, according to an embodiment of the present disclosure (hereinafter, referred to as the present embodiment), a latex agglutination reaction sensitizer, a latex agglutination reagent, and a method for measuring a target substance in a specimen by a latex agglutination method using the same will be described, but the present disclosure is not limited thereto.

The latex agglutination reagent of the present embodiment is used for measuring a target substance in a specimen by a latex agglutination method using an insoluble carrier that carries an antibody or an antigen. In the present specification, the latex agglutination method is an immunoassay method based on an agglutination reaction, and examples thereof include an immunoassay method called a latex agglutination Turbidimetric immunoassay, a Turbidimetric immunoassay, an immuno-nephelometry (Nephelometric immunoassay), or the like.

### (Polyoxazoline as Sensitizer)

The latex agglutination reagent of the present embodiment has a feature to contain at least polyoxazoline that functions as a latex agglutination reaction sensitizer, and for example, polyoxazoline can be contained in various solutions used in the latex agglutination method to prepare the latex agglutination reagent.

In the latex agglutination reagent of the present embodiment, the mechanism by which polyoxazoline has a sensitization action of an agglutination reaction is considered to be depletion flocculation. The depletion flocculation is interparticle agglutination due to the osmotic pressure acting between particles, which occurs as a result of a dissolved polymer with finite extensibility being removed from gaps of particles when the particles approach each other. In order to effectively cause the depletion flocculation, it is demanded that the polymer has a weak interaction with a medium and media in an aqueous solution and has an appropriate size in the aqueous solution. In the present embodiment, as will be described later, polyoxazoline has a weak interaction with salts and ions, and also has a weak interaction with proteins and insoluble carriers. It was also found that polyoxazoline effectively functions as a sensitizer for the latex agglutination reagent since the viscosity of the solution does not easily increase.

The polyoxazoline in the present embodiment is a polymer containing a repeating unit represented by the following General Formula (1). In the present embodiment, the polyoxazoline is a general term for poly(N-acylethyleneimine). According to a preferable aspect, the polyoxazoline used according to the present embodiment is a polymer of 2-alkyl-2-oxazoline and may be referred to as poly(2-oxazoline).

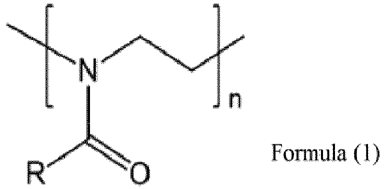

In Formula (1), R formed on a polymer side-chain is selected from a hydrogen atom or a methyl group, an ethyl group, a propyl group, or a combination thereof. Examples thereof include poly(2-methyl-2-oxazoline), poly(2-ethyl-2-oxazoline), poly(2-isopropyl-2-oxazoline), poly(2-normalpropyl-2-oxazoline), and the like. Hydrophobicity of R increases as an alkyl chain is longer. Polyoxazolines with longer alkyl side-chains may not be preferable as hydrophobicity of the polymer increases and solubility of the polymer in water is thus reduced. In Formula (1), R formed on the polymer side-chain can be a methyl group or an ethyl group, and can be more preferably an ethyl group. Examples of preferable polymers include poly(2-methyl-2-oxazoline) and poly(2-ethyl-2-oxazoline), and in particular, poly(2-ethyl-2-oxazoline) with a good balance of water-soluble and hydrophobic represented by the following General Formula (2) is good.

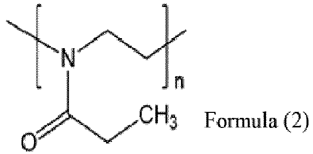

In Formulae (1) and (2), n indicating the degree of polymerization of the polymer (also referred to as the number of repeating units) is an integer of 100 or greater, and preferably 100 or greater to 100,000 or smaller. In a case of a homopolymer of the poly(2-ethyl-2-oxazoline) of Formula (2), a weight average molecular weight of the polymer is about 10,000 or greater to 1,000,000 or smaller within the range of the degree of polymerization of the polymer. When n indicating the degree of polymerization of the polymer is smaller than 100, the sensitization action is lowered since the molecular weight is small. By contrast, when n is greater than 100,000, the molecular weight is increased and the viscosity of the solution is too high, which is not preferable.

The polyoxazoline in the present embodiment may be a homopolymer or a copolymer as long as the polyoxazoline contains a repeating unit represented by General Formula (1). Specific examples of the copolymer include a copolymer of polyoxazoline and polyethylene glycol, a copolymer of polyoxazoline and polylactic acid, a copolymer of polyoxazoline and polyacrylic acid, and the like.

These polyoxazolines can be used alone or in combination in the latex agglutination reagent and the method for measuring a target substance using the same of the present embodiment. The latex agglutination reagent can contain poly(2-ethyl-2-oxazoline) of Formula (2), and the method is preferably a method for measuring a target substance using the same.

The polyoxazoline can be produced by, for example, polymerization of an oxazoline monomer with a known polymerization reaction. For example, under appropriate conditions, the polyoxazoline can be synthesized by cationic ring-opening polymerization of 2-oxazoline. As an example of a method for synthesizing polyoxazoline, poly(2-ethyl-2-oxazoline) (hereinafter, may be abbreviated as "POX") will be described as an example. Methyl tosylate (initiator) and 2-ethyl-2-oxazoline (monomer) are added in acetonitrile under a dry argon atmosphere, and cationic ring-opening polymerization is carried out. The reaction is carried out at a temperature of 40°C for about 10 days, and thereafter, sodium hydroxide is added to stop the reaction in order to introduce a hydroxyl group (terminating agent) to the end of the polymer. The reaction solution is dialyzed against water to be purified and dried under reduced pressure to obtain a solid content, and therefore, a poly(2-ethyl-2-oxazoline) homopolymer can be synthesized.

A terminal structure of the polyoxazoline is not particularly limited, but for example, one end is a functional group derived from a polymerization initiator and the other end is a functional group derived from a polymerization terminating agent. In the cationic ring-opening polymerization, an electrophilic polymerization initiator and a nucleophilic polymerization terminating agent are appropriately selected, so that any functional group can be introduced onto both ends of the polymer chain. Examples of the electrophilic polymerization initiator include alkyl halides, acid halides, polyfunctional tosylate, polyfunctional triflate, polyfunctional nosylate, and the like. Examples of the nucleophilic polymerization terminating agent include hydroxy oxides, amines, carboxylates, and the like. Examples of the terminal structure of the polyoxazoline include a methyl group and a hydroxyl group.

The range of the weight average molecular weight of the polyoxazoline is not particularly limited, but preferably 10,000 or greater to 1,000,000 or smaller. The degree of polymerization of the polymer is as described above, and it is necessary that the polymer is dispersed to some extent in water in order to exert the sensitization action, while the viscosity of the solution is not too high, as a latex agglutination reagent. Therefore, in a case where polyoxazoline having a weight average molecular weight of 50,000, even more preferably having a weight average molecular weight of at least 200,000, and most preferably having a weight average molecular weight of at least 400,000 is used, a high sensitization effect is observed. When the weight average molecular weight is smaller than 10,000, it is difficult to obtain a sufficient sensitization action since the molecular size in water is small. When the weight average molecular weight is greater than 1,000,000, the viscosity of the solution is high, so that the handling is inferior and a sufficient effect cannot be obtained. The weight average molecular weight of the polyoxazoline is determined based on gel permeation chromatography (GPC) and a viscosity measurement.

### (Latex Agglutination Reagent)

The latex agglutination reagent of the present embodiment has a feature to contain at least polyoxazoline that functions as a sensitizer, and for example, polyoxazoline can be contained in aqueous media used in the latex agglutination method to prepare the latex agglutination reagent.

A concentration of the polyoxazoline in the latex agglutination reagent of the present embodiment is preferably 0.01 w/v% or more to 2.0 w/v% or less from the viewpoint of solubility. When the concentration of the polyoxazoline is in this range, the viscosity of the solution is low and the solution is easy to handle. When the concentration of the polyoxazoline is too low, it is difficult to obtain a sufficient sensitization effect. As will be described later, in the measurement method based on the latex agglutination method of the present embodiment, the polyoxazoline according to the present embodiment is added and used to be usually 0.005 w/v% or more to 1.9 w/v% or less as the final concentration in the reaction solution, preferably 0.1 w/v% or more to 1.9 w/v% or less, and more preferably 0.15 w/v% or more to 1.9 w/v% or less. The concentration can be changed depending on types of the target substance to be measured and the insoluble carrier.

The latex agglutination reagent of the present embodiment is an aqueous solution of polyoxazoline, and examples of aqueous media include various buffers such as purified water, a phosphate buffer, a glycine buffer, a Good's buffer, a tris buffer, an ammonia buffer, and the like. In particular, a phosphate buffer and a Good's buffer are preferably used.

The latex agglutination reagent of the present embodiment contains polyoxazoline in an aqueous medium, but other additives such as salts, bovine serum albumin (BSA), surfactants, and immunoglobulins may be included. In particular, in order to adjust the osmotic pressure of the solution and stabilize proteins, sodium chloride is preferably added.

### (Insoluble Carrier)

As the insoluble carrier that can be suitably used with the latex agglutination reagent of the present embodiment, any insoluble carrier used in a normal latex agglutination reaction can be used, and a granular carrier (hereinafter, abbreviated as "particle") can be used. Examples of materials for the insoluble carrier include polystyrene, a styrene-methacrylic acid copolymer, a styrene-glycidyl (meth)acrylate copolymer, an acrylic acid polymer, polymers such as agarose and dextran, inorganic substances such as silica and alumina, and metals such as gold colloids. The synthesis and preparation of the insoluble carrier can be carried out according to a known method.

Examples of suitable carriers include latex particles. Here, the latex particles are polymer particles capable of immobilizing antigens or antibodies against a target substance, and mean particles used for the latex agglutination reaction. Preferable examples of the latex particles include polystyrene particles, polystyrene particles containing siloxane, polystyrene particles containing polyglycidyl (meth)acrylate (hereinafter, may be abbreviated as "SG particles"), and the like. These particles have advantages such as the fact that nano-sized particles can be obtained relatively easily and a surface of each particle can be chemically modified according to the purpose, and are carriers that can be suitably used with the latex agglutination reagent of the present embodiment.

Among these carriers, as will be described later in Examples, polystyrene particles containing siloxane and SG particles, each of which has a chemically hydrophilic particle surface, can highly suppress non-specific adsorption. Therefore, there is almost no interaction between the polyoxazoline according to the present embodiment and the particles, and it is the most ideal combination of the particles and the sensitizer in promoting agglutination by the depletion effect.

As a specific effect, in a case where polystyrene particles containing siloxane or SG particles are used as carriers, it is advantageous that the sensitivity can be increased by an increase in a concentration of the polyoxazoline, and the measurement accuracy is further improved since the polyoxazoline exhibits a low viscosity, and the polyoxazoline is not adsorbed on the particles.

The size of the insoluble carrier preferably has a major axis diameter of 0.01 µm or greater to 10 µm or smaller, and more preferably has 0.05 µm or greater and 1 µm or smaller. By setting the size of the insoluble carrier to 0.05 µm or greater, absorbance in the visible range observed by the latex agglutination measurement is within an appropriate range. By setting the size of the insoluble carrier to 1 µm or smaller, dispersion stability in the solution is improved, and the absorbance in the visible range is within an appropriate range. From the viewpoint of reactivity and dispersion stability, an insoluble carrier of 0.05 µm or greater to 0.40 µm or smaller is preferably used. As described above, an example of a suitable insoluble carrier is particles. Therefore, a suitable example of the size can be represented in terms of average particle size. As the average particle size, a volume average particle size or the like measured by the measurement of scattered particle size distribution is used. The average particle size of the particles as the insoluble carrier is preferably 0.05 µm or greater to 1 µm or smaller.

The amount of the insoluble carrier used can also be adjusted as appropriate, depending on its size, and the concentration of the insoluble carrier in the solution during the agglutination reaction is the amount of 0.01 w/v% or more to 0.5 w/v% or less, or 0.02 w/v% or more to 0.3 w/v% or less from the viewpoint of reactivity and solution turbidity.

The insoluble carrier is preferably an insoluble carrier on which antigens or antibodies are immobilized. Each of antigens or antibodies may be immobilized on the insoluble carrier by a chemical bond or physical adsorption, but each of antigens or antibodies is preferably immobilized by a chemical bond. Immobilization of antigens or antibodies may be carried out according to a method in the related art, but a functional group of the insoluble carrier can be reacted with a primary amine, a secondary amine, a carboxyl group, a thiol group or the like contained in the antigens or antibodies. Examples of the functional group of the insoluble carrier include a carboxyl group, an amino group, an aldehyde group, an epoxy group, a thiol group, a maleimide group, and the like. The insoluble carrier may have only one functional group in these functional groups, or may have two or more functional groups.

In addition, instead of the antibodies, an antigen-binding fragment such as Fab, F(ab')2, F(ab'), Fv, scFv can also be used.

In addition to the configuration in which each of the antigens or antibodies is immobilized on the insoluble carrier, a configuration in which an appropriate substance is bound according to the target substance may be adopted. For example, an insoluble carrier may be bound with an enzyme protein or a substrate thereof, a signal substance such as a hormone or a neurotransmitter, or a receptor thereof, a nucleic acid, or a substance such as avidin or biotin.

### (Target Substance)

The latex agglutination reagent of the present embodiment is used for the measurement of a target substance, and the target substance may be a substance that can be measured by an immune reaction, and examples thereof include substances such as antigens and antibodies. Examples thereof include C-reactive protein (CRP), ferritin, albumin, immunoglobulin G (IgG), immunoglobulin A (IgA), immunoglobulin M (IgM), immunoglobulin E (IgE), α-feto protein (AFP), carcinoembryonic antigen (CEA), prostate-specific antigen (PSA), KL-6, pepsinogen (PG), rheumatoid factor (RF), lipoprotein, insulin, infection-related antigens and antibodies thereof, hormones, vitamins, antibiotics, and the like, but the latex agglutination reagent of the present embodiment is not limited thereto.

### (Preparation Example of Latex Agglutination Reagent)

The polyoxazoline according to the present embodiment can be contained in various reagents used in immunoassay to prepare a latex agglutination reagent having a high sensitization action.

An example of the latex agglutination reagent of the present embodiment is illustrated.

### (1 Liquid Type Latex Agglutination Reagent)

Polyoxazoline is dissolved in phosphate-buffered saline (may be abbreviated as PBS).

Polystyrene particles (insoluble carriers) sensitized to antibodies against the target substance are added to this polyoxazoline solution, so that the resultant solution can be obtained as an example of the embodiment of the latex agglutination reagent of the present disclosure. A sample of serum, plasma, or the like (hereinafter, may be abbreviated as a specimen) is added to a 1 liquid type latex agglutination reagent, and the turbidity is measured with a spectrophotometer. Since the polystyrene particles remain in a dispersed state in a case where the sample does not contain the target substance, there is no change in turbidity over time. By contrast, in a case where the sample contains the target substance, the polystyrene particles agglomerate by an antigen-antibody reaction with the target substance. Due to this agglutination, the turbidity changes over time. That is, the turbidity increases. Since a calibration curve (a graph illustrating the relation between a turbidity and a concentration of the target substance) is obtained by the measurement of the turbidity using a standard solution of the target substance of which a concentration is known, the target substance contained in the specimen can be quantified from an increase in the turbidity of the sample. The latex agglutination reagent of the present embodiment may contain various additives. In general, the latex agglutination reagent may contain a pH buffer (for example, tris buffer, phosphate buffer, borate buffer, Good's buffer, or the like), proteins such as albumin and globulin, amino acids, surfactants, preservatives, and the like.

### (Latex Agglutination Reagent Kit)

One of the forms of the present disclosure is a latex agglutination reagent kit including at least the latex agglutination reagent that contains polyoxazoline as a constituent component, and as a specific example, the latex agglutination reagent is composed of at least two or more solutions. Examples of these solutions include a diluent of the specimen, a dispersion of the insoluble carriers, a diluent of the dispersion of the insoluble carriers, a standard solution (a solution containing the target substance with a known concentration) for acquiring a calibration curve, and a control for confirming the measurement accuracy, and the like. An example of the kit is a latex agglutination reagent kit including at least a first reagent composed of a latex agglutination reagent containing polyoxazoline and a second reagent containing an insoluble carrier that carries an antibody or an antigen. The first reagent composed of a latex agglutination reagent containing polyoxazoline may be referred to as a specimen diluent. Polyoxazoline may be contained in the second reagent that contains an insoluble carrier, while the polyoxazoline is not contained this specimen diluent. Usually, from the viewpoint of storage stability, polyoxazoline is preferably contained in the first reagent or the specimen diluent.

In the measurement of the target substance by the latex agglutination method, for example, a specimen is added to the first reagent composed of the latex agglutination reagent containing polyoxazoline, and the second reagent containing insoluble carriers that carry antibodies or antigens is then mixed to this solution. In this case, the latex agglutination reaction is preferably carried out within the range in which a polyoxazoline concentration is 0.005 w/v% or more to 1.0 w/v% or less, and the amount of the specimen, the amount of the first reagent, and the amount of the second reagent are set, and thereafter, the polyoxazoline concentration in the first reagent may be set. During the reaction of latex agglutination, the viscosity of the solution is low, the solution is easy to handle, and a sufficient sensitization effect can be obtained within the range of the polyoxazoline concentration. The concentration can be appropriately set depending on types of the target substance to be measured and the insoluble carrier.

### (Method for Measuring Target Substance in Specimen)

The present embodiment is a method for measuring a target substance in a specimen by a latex agglutination method using an insoluble carrier that carries an antibody or an antigen, wherein in the method for measuring a target substance in a specimen, the latex agglutination reagent or the latex agglutination reagent kit according to the present embodiment is used. Here, the measurement includes the meaning of qualitative measurement and quantitative measurement.

In the method for measuring the target substance in the specimen, a preferable form is a latex agglutination method using latex particles carrying antibodies or antigens.

In an example of the latex agglutination method, a specimen (serum or the like) that may contain a target substance reacts with a latex agglutination reagent that contains the latex particles carrying antibodies against the target substance to produce agglomerates by this antigen-antibody reaction, and this agglutination of the agglomerates is visually or optically detected, thereby the target substance capable of being qualitatively and quantitatively measured.

In another example of the latex agglutination method, a specimen that may contain the target substance is mixed with a specimen diluent (a first reagent of the latex agglutination reagent). Next, a latex particle dispersion (a second reagent of the latex agglutination reagent) that contains latex particles on which antibodies or antigens against the target substance are immobilized is further mixed. In a case where the target substance is present when the first reagent and the second reagent are mixed, the latex particles agglomerate as a result of the antigen-antibody reaction. Therefore, the target substance can be qualitatively and quantitatively measured by visual or optical detection of this agglutination.

As the latex agglutination method, as described above, the latex agglutination method in which the polyoxazoline concentration during the reaction is within the range of 0.005 w/v% or more to 1.0 w/v% or less is preferably performed. In a case where the polyoxazoline concentration is within this range, the target substance can be measured with high sensitivity and high measurement accuracy, in the measurement of the target substance in the specimen by the latex agglutination method.

The latex agglutination method is generally performed within the reaction temperature range of 4°C or higher to 50°C or lower. In a case where the temperature is low, the reactivity of the antigen-antibody decreases, and in a case where the temperature is high, the stability of the immune complex of the antigen-antibody decreases. Therefore, the reaction temperature is preferably 10°C or higher to 40°C or lower, and particularly preferably 30°C or higher to 40°C or lower. The latex agglutination method is generally performed within the reaction time range of 0 minutes or longer to 60 minutes or shorter. The range is preferably 1 minute or longer to 30 minutes or shorter.

Agglomerates produced by the antigen-antibody reaction can be visually or optically detected, thereby measuring the target substance. That is, the target substance can be measured qualitatively and quantitatively. In this detection step, the agglutination formation may be visually observed, but from the viewpoint of detection reproducibility and throughput, the degree of agglutination is preferably measured using an optical device. As the optical device, a device capable of measuring scattered light or transmitted light is preferably used, and a spectrophotometer or the like can be used.

In the latex agglutination method of the present embodiment, the insoluble carriers are preferably polystyrene particles, polystyrene particles containing siloxane, or SG particles. As the latex agglutination method, the polyoxazoline concentration during the reaction can also be changed depending on the insoluble carriers used.

The target substance to be measured by the latex agglutination method of the present embodiment may be any substance as long as the substance can be measured by an immune reaction, and examples thereof include substances such as antigens and antibodies. Examples thereof include C-reactive protein (CRP), ferritin, albumin, immunoglobulin G (IgG), immunoglobulin A (IgA), immunoglobulin M (IgM), immunoglobulin E (IgE), α-feto protein (AFP), carcinoembryonic antigen (CEA), prostate-specific antigen (PSA), KL-6, pepsinogen (PG), rheumatoid factor (RF), lipoprotein, insulin, infection-related antigens and antibodies thereof, hormones, vitamins, antibiotics, and the like, but the latex agglutination reagent of the present embodiment is not limited thereto.

As the latex agglutination method, the polyoxazoline concentration during the reaction can also be changed depending on the target substance.

### [Examples]

Hereinafter, as Examples of the present disclosure (hereinafter, referred to as the present example), a latex agglutination reagent kit composed of two liquids will be described, but the present disclosure is not limited thereto.

### <Example 1: Preparation Example of First Reagent of Latex Agglutination Reagent Kit>

An example of a latex agglutination reagent kit composed of two liquids includes a first reagent for diluting a specimen and a second reagent containing an antibody-sensitized latex. Here, a preparation example of the first reagent is illustrated.

### <Example 1-1: Preparation of Latex Agglutination Reagent (First Reagent) Containing Poly(2-ethyl-2-oxazoline)>

Poly(2-ethyl-2-oxazoline) (average molecular weight of 400,000, sigma-aldrich) was dissolved in a PBS solution (pH 7.4). Hereinafter, poly(2-ethyl-2-oxazoline) may be abbreviated as "POX". The POX concentration was adjusted to be a concentration of 0.5 w/v% to obtain a latex agglutination reagent (first reagent) of the present example. Hereinafter, this first reagent will be referred to as R1 + POX.

### (Comparative Example 1-1: Preparation of Latex Agglutination Reagent in Related Art)

For comparison, a first reagent (that is, a PBS solution) containing no poly(2-ethyl-2-oxazoline) was prepared in the same manner as in Example 1-1. This will be referred to as R1.

PEG (average molecular weight of 400,000, Sigma-Aldrich), which is a nonionic polymer widely used as a sensitizer, and sodium alginate (80-120, Wako Pure Chemical Industries, Ltd.) (SA), which is an anionic polymer, were dissolved in PBS to have a concentration of 0.5 w/v%, thereby preparing each of R1 + PEG and R1 + SA as a first reagent for comparison.

### <Example 1-2: Viscosity Measurement of Latex Agglutination Reagent (First Reagent)>

The viscosity of the latex agglutination reagent (first reagent) R1 + POX of the present example was measured. The viscosity was measured with an E-type rotational viscometer (RE-80, Toki Sangyo Co., Ltd.). The results are illustrated in Table 1.

### (Comparative Example 1-2)

For comparison, the viscosities of R1 + PEG and R1 + SA were measured. The results are illustrated in Table 1.

**[Table 1]**

| | First reagent | Concentration of sensitizer w/v% | Viscosity mPa·s |
|---|---|---|---|
| Example 1-2 | R1+POX | 0.5 | 1.45 |
| Comparative Example 1-1 | R1 | 0.0 | 0.94 |
| Comparative Example 1-2 | R1+PEG | 0.5 | 4.10 |
| Comparative Example 1-2 | R1+SA | 0.5 | 8.00 |

From the results in Table 1, in the polymer solutions having the same concentration as each other, R1 + POX had a higher viscosity of R1 of Comparative Example, but had a lower viscosity of R1 + PEG and R1 + SA that are solutions containing a coagulant in the related art. It was found that the poly(2-ethyl-2-oxazoline) of the present example had similar properties and did not increase the viscosity of the solution as compared with PEG that is a polymer having a similar molecular weight. Although SA had a particularly high viscosity, it is estimated that the molecular weight is several million, so that it is considered that the effect of the molecular weight is also included.

### <Example 1-3: Viscosity Measurement of Latex Agglutination Reagent (First Reagent)>

The viscosity of the latex agglutination reagent (first reagent) R1 + POX of the present example in a case where the POX concentration was changed was measured. The viscosity was measured with an E-type rotational viscometer (RE-80, Toki Sangyo Co., Ltd.). The results are illustrated in Table 2.

### (Comparative Example 1-3)

For comparison, the viscosities of R1 + PEG and R1 + SA in a case where the polymer concentration was changed were measured in the same manner as in Example 1-3. The results are illustrated in Table 2, and "-" indicates "unmeasured".

**[Table 2]**

| | Viscosity of solution (mPa·s) | | |
|---|---|---|---|
| | Example 1-3 | Comparative Example 1-3 | |
| Polymer concentration (w/v%) | R1+POX | R1+PEG | R1+SA |
| 0.000 | 0.94 | 0.94 | 0.94 |
| 0.050 | - | - | - |
| 0.100 | - | 1.45 | - |
| 0.200 | - | 1.90 | 2.65 |
| 0.250 | - | 2.29 | 3.14 |
| 0.375 | - | 3.22 | 6.01 |
| 0.500 | 1.45 | 4.10 | 8.00 |
| 1.000 | 2.12 | | |
| 1.500 | 2.43 | | |
| 1.750 | 3.21 | | |
| 2.000 | 3.93 | | |

From the results in Table 2, it was found that the viscosity of R1 + POX increased as the polymer concentration increased, but the increase in viscosity gradually increased as compared with Comparative Examples of R1 + PEG and R1 + SA. The poly(2-ethyl-2-oxazoline) of the present example has an advantage that the viscosity of the solution can be adjusted more gradually than PEG and SA, which are similar polymers.

### <Example 2: Preparation Example of Second Reagent of Latex Agglutination Reagent Kit>

An example of a latex agglutination reagent kit composed of two liquids includes a first reagent for diluting a specimen and a second reagent containing an antibody-sensitized latex. Here, a preparation example of the second reagent is illustrated.

### (Synthesis Example 2-1: Synthesis of SG particles)

12.0 g of styrene (St: Kishida Chemical Co., Ltd.), 17.9 g of glycidyl methacrylate (GMA: Tokyo Chemical Industry Co., Ltd.), 0.45 g of divinylbenzene (DVB: Kishida Chemical Co., Ltd.) 2168.6 g of ion-exchanged water were weighed and charged into a 2 L four-necked separable flask to produce a mixed solution, this mixed solution was held at 70°C while stirring at 200 rpm, and nitrogen flow was carried out at a flow rate of 200 ml/min, thereby deoxidizing the inside of the above described four-necked separable flask. Next, a dissolving solution prepared such that 1.13 g of V-50 (FUJIFILM Wako Pure Chemical Corporation), which was separately adjusted, was dissolved in 30 g of ion-exchanged water was added to the mixed solution, thereby starting soap-free emulsion polymerization. Two hours after the start of the polymerization, 3.1 g of GMA was added to the above described four-necked separable flask, and the mixture was held at 70°C for 22 hours while being stirred at 200 rpm, thereby obtaining an aqueous dispersion that contains a granular copolymer A. After slowly cooling the dispersion to room temperature, a part thereof was sampled, and a polymerization conversion was evaluated using proton NMR, gas chromatography, and gel permeation chromatography. As a result, it was confirmed that the polymerization conversion was substantially 100%. The dry particle size of the granular copolymer A was 151.4 nm, and the particle size in water was 160.2 nm. The granular copolymer A was diluted by ultrafiltration concentration or ion-exchanged water to be an aqueous dispersion of 2.5 w/v%, and stored at 4°C under the light-shielding condition. Next, a 2.5 w/v% aqueous dispersion containing 24 g of the granular copolymer A, 3.3 g of ion-exchanged water, 40 mg (0.26 mmol) of mercapto succinic acid (FUJIFILM Wako Pure Chemical Corporation), and 0.214 mL (2.34 mmol) of 3-mercapto-1,2-propanediol (FUJIFILM Wako Pure Chemical Corporation) were weighed and charged into a 100 ml round bottom flask, and triethylamine (Kishida Chemical Co., Ltd.) was added to adjust pH = 10. Next, the contents of the above described round-bottom flask were heated to 70°C while being stirred at 200 rpm, and held in this state for further 18 hours to obtain a dispersion of SG particles (hereinafter, abbreviated as a "latex A particles"). The latex A particles were separated from the above described dispersion by a centrifuge, and the operation of redispersing the latex A particles in ion-exchanged water was repeated 8 times to purify the latex A particles, and finally, the latex A particles were adjusted to be 1.0 w/v% and stored to be in a state of an aqueous dispersion. The storage condition was 4°C under the light-shielding condition. The particle size of the latex A particles in water was 202.0 nm.

### (Synthesis Example 2-2: Synthesis of Polystyrene Particles Containing Siloxane)

157 g of 50 mM 2-morpholinoethanesulfonic acid (MES, manufactured by Tokyo Chemical Industry Co., Ltd.) buffer (pH 7.0) and 1.3 g of polyvinylpyrrolidone K-30 (molecular weight 40,000, manufactured by Kishida Chemical Co., Ltd.) were mixed. Next, 4 g of 3-methacryloxypropyltrimethoxysilane (LS-3380 manufactured by Shin-Etsu Chemical Co., Ltd.) and 13 g of styrene (manufactured by Kishida Chemical Co., Ltd.) were added, and the mixture was stirred for 10 minutes while blowing nitrogen at room temperature. The emulsion in the flask was then heated to 70°C in an oil bath. 0.5 g of potassium peroxodisulfate (manufactured by Wako Pure Chemical Industries, Ltd.) was dissolved in ion-exchanged water, and the obtained solution was added to the emulsion that has been heated to 70°C. After stirring at 70°C for 7 hours, heating was stopped and the stirring was performed overnight to obtain a dispersion of a polystyrene particle precursors containing siloxane. Next, the dispersion of the polystyrene particle precursors containing siloxane was centrifuged to collect the polystyrene particle precursors containing siloxane, and the supernatant was discarded. The polystyrene particle precursors containing siloxane collected were redispersed in ion-exchanged water and then washed by repeating the operation of centrifuging a plurality of times. In a case where the average particle size of the polystyrene particle precursors containing siloxane thus obtained was evaluated by dynamic light scattering using a particle size analyzer (trade name: Zetasizer, manufactured by Malvern Panalytical Ltd), the average particle size was 203 nm. Next, 0.4 g of polystyrene particle precursors containing siloxane was mixed with Tween (registered trademark) 20 (manufactured by Tokyo Chemical Industry Co., Ltd.) having a final concentration of 1 w/v% and 40 mL of ion-exchanged water. After stirring this mixed solution at room temperature for 15 minutes, 0.04 mL of X-12-1135 (manufactured by Shin-Etsu Chemical Co., Ltd.), which is a silane coupling agent having a carboxyl group, was added and stirred overnight at room temperature. Subsequently, this dispersion was centrifuged and the supernatant was discarded. The particles collected were redispersed in ion-exchanged water and then washed by repeating the operation of centrifuging a plurality of times. The obtained particles were polystyrene latex particles containing siloxane (hereinafter abbreviated as "latex B particles").

### <Example 2-1: Preparation Example of Second Reagent of Latex Agglutination Reagent Kit>

The carboxyl groups of the latex A particles and the latex B particles obtained in Synthesis Example 2-1 and Synthesis Example 2-2 were activated by a known method using WSC/NHS (water-soluble carbodiimide/N-hydroxysuccinimide). 0.05 mL of a 10 w/v% aqueous solution of the activated latex A particles and latex B particles and 0. 05 mL of a 50 mM MES buffer (pH 5.0) containing 1 mg/mL of anti-human CRP polyclonal antibodies (manufactured by Oriental Yeast Co., Ltd.) were mixed and reacted with each other at room temperature for 1 hour. Thereafter, the antibody-immobilized particles obtained by washing of the solution with centrifugation were referred to as antibody-sensitized latex A particles and antibody-sensitized latex B particles, respectively, and the aqueous dispersion thereof was referred to as an antibody-sensitized latex A particle dispersion and an antibody-sensitized latex B particle dispersion, and these were used as the second reagent. The antibody-sensitized latex A particle dispersion and the antibody-sensitized latex B particle dispersion were referred to as R2-A and R2-B, respectively. In addition, the preparation result prepared in the same manner as the above description except that commercially available polystyrene latex C particles having a particle size of 200 nm (IMMUTEX (registered trademark) P0113 manufactured by JSR Corporation) were used and post-coating was performed with bovine serum albumin (BSA) was used as an antibody-sensitized latex C particle dispersion, that is, a second reagent. This is referred to as R2-C.

### (Sample)

CRP-free human serum (manufactured by Oriental Yeast Co., Ltd.) was used to confirm non-specific reactions to human serum. A CRP solution was used as an antigen solution for sensitivity measurement to confirm the sensitization effect. A CRP solution having a CRP concentration of 0.5 mg/dL was used.

### <Example 2-2: Evaluation 1 of Non-specific Reactivity and Sensitization Effect by Latex Agglutination Measurement>

The non-specific reactivity for the latex agglutination reagent of the present example was confirmed by latex agglutination measurement using human serum. 1 µL of CRP-free human serum in a sample was added to 50 µL of the first reagent (R1 + POX) of the present example obtained in Example 1-1. This was charged in a spectrophotometer measurement cell (optical path length of 10 mm) and held at 37°C for 5 minutes. Next, 50 µL of the second reagent (R2-A solution) of the present example was added to 51 µL of the R1 solution and mixed sufficiently. Thereafter, the absorbance at 572 nm was measured using a spectrophotometer (referred to as absorbance at 0 minutes). After 5 minutes, the absorbance at 572 nm was measured again (referred to as absorbance after 5 minutes). The difference between the absorbances at the two obtained points ((absorbance after 5 minutes) - (absorbance at 0 minutes)) is determined, and a value obtained by multiplying the determined difference by 10,000 is referred to as the amount of change in absorbance. In a case where there is no non-specific reaction, the value will be close to zero. In a case where a non-specific reaction occurs, the value will be increased. Here, it is defined that a non-specific reaction has occurred in a case where the amount of change in absorbance is 100 or greater. The non-specific reaction of R1 + POX was evaluated according to the above described measurement method. The results are illustrated in Tables 3 and 4. Here, the non-specific reactivity means the degree of the above described non-specific reaction and is represented by the amount of change in absorbance.

The sensitization effect of the latex agglutination reagent of the present example was confirmed by the latex agglutination measurement of CRP. 1 µL of the CRP solution having a CRP concentration of 0.5 mg/dL of the above described sample was added to 50 µL of the above described first reagent (R1 + POX) of the present example obtained in Example 1-1. This was charged in a spectrophotometer measurement cell (optical path length of 10 mm) and held at 37°C for 5 minutes. Next, 50 µL of the second reagent (R2-A solution) of the present example was added to 51 µL of the R1 solution and mixed sufficiently. Thereafter, the absorbance at 572 nm was measured using a spectrophotometer (referred to as absorbance at 0 minutes). After 5 minutes, the absorbance at 572 nm was measured again (referred to as absorbance after 5 minutes). The difference between the absorbances at the two obtained points ((absorbance after 5 minutes) - (absorbance at 0 minutes)) is determined, and a value obtained by multiplying the determined difference by 10,000 is referred to as the amount of change in absorbance. In a case where particle agglutination occurs due to CRP and antibody-antigen reaction, the amount of change in absorbance increases. The higher the amount of change in absorbance, the higher the sensitivity. The CRP measurement sensitivity (a CRP concentration of 0.5 mg/dL) of R1 + POX was evaluated according to the above described measurement method. The results are illustrated in Table 3. Here, the CRP measurement sensitivity means the degree of particle agglutination due to the CRP and antibody-antigen reaction, and is represented by the amount of change in absorbance.

### (Comparative Example 2-2)

For comparison, the non-specific reactivity and sensitization effect of R1 + PEG and R1 + SA were evaluated in the same manner as in Example 2-2. The results are illustrated in Table 3.

**[Table 3]**

| | | Amount of change in absorbance | | | | | |
|---|---|---|---|---|---|---|---|
| | | Example 2-2 | | Comparative Example 2-2 | | | |
| | | R1+POX | | R1+PEG | | R1+SA | |
| Polymer concentration of first reagent (w/v%) | Polymer concentration during reaction (w/v%) | Non-specific reactivity | CRP measurement sensitivity | Non-specific reactivity | CRP measurement sensitivity | Non-specific reactivity | CRP measurement sensitivity |
| 0 | 0.000 | 30 | 900 | 30 | 900 | 30 | 900 |
| 0.05 | 0.025 | -10 | 1190 | -20 | 1310 | 40 | 1810 |
| 0.1 | 0.050 | -70 | 1530 | 40 | 1600 | 40 | 2660 |
| 0.2 | 0.099 | -40 | 1690 | -30 | 2120 | 20 | 7030 |
| 0.25 | 0.124 | 10 | 1790 | 50 | 3310 | 220 | 13810 |
| 0.375 | 0.186 | -50 | 2090 | 120 | 4830 | 16400 | Unmeasurable^{∗} |
| 0.5 | 0.248 | 20 | 2540 | 290 | 6170 | Unmeasurable^{∗} | Unmeasurable^{∗} |
| 1 | 0.495 | -30 | 4230 | 18540 | 16960 | | |
| 1.5 | 0.743 | -50 | 8570 | | | | |
| 1.75 | 0.866 | 40 | 10600 | | | | |
| 2 | 0.990 | 270 | 10390 | | | | |
| 2.5 | 1.238 | 2850 | 15610 | | | | |

The evaluation results of the non-specific reactivity and the sensitization effect by latex agglutination measurement, in a case where a R2-A solution was used as the second reagent, that is, SG particles are used as insoluble carriers, and the polymer concentration of the first reagent is changed, are illustrated in Table 3.

The sensitivity of R1 + POX was higher than that of R1 (Comparative Example) containing no sensitizer, and the CRP measurement sensitivity was improved depending on the POX concentration. That is, it was confirmed that POX functions as a sensitizer for the latex agglutination reagent. Furthermore, R1 + POX had very low non-specific reactivity to human serum at concentrations up to 0.866%. By contrast, R1 + PEG and R1 + SA of Comparative Example had the high sensitivity and functioned as sensitizers, while a non-specific reaction occurred. As illustrated by ^{∗} in the table, specifically, in a case where the polymer concentration of R1 + SA during the reaction is 0.186% or more, agglutination occurred rapidly when the absorbance at 0 minutes was measured, and the absorbance was 3.0 or higher (range over), which is the upper limit of measurement by the spectrophotometer, so that the measurement could not be performed (indicated in the table as unmeasurable). Agglutination in PEG and alginic acid may occur due to salts, and there are problems such as bubble generation during stirring and reaction reproducibility since the viscosity of the solution is high. When comparing with the result of R1 + PEG and the result of R1 + SA of Comparative Example 2-2, assuming that the latex agglutination reagent of the present example containing poly(2-ethyl-2-oxazoline) has an amount of change in absorbance of 100 or smaller in a case where the non-specific reaction does not occur, the maximum sensitivity is 10,600, and the amount of change in absorbance can be increased. The maximum sensitivity of Comparative Examples was 3,310 for PEG and 7,030 for SA in a case where the non-specific reaction does not occurred.

From the above results, in a case of using the latex agglutination reagent containing poly(2-ethyl-2-oxazoline) of the present example when SG particles are used as the insoluble carriers, the sensitization effect could be expressed while suppressing the non-specific reaction, and the latex agglutination could be measured with high sensitivity.

### <Example 2-3: Evaluation 2 of Non-specific Reactivity and Sensitization Effect by Latex Agglutination Measurement>

Similar to Example 2-2, the non-specific reactivity and the sensitization effect were evaluated by the latex agglutination measurement when a R2-C solution was used as the second reagent, that is, polystyrene latex particles were used as insoluble carriers.

The results are illustrated in Table 4.

### (Comparative Example 2-3)

For comparison, the non-specific reactivity and sensitization effect of R1 + PEG and R1 + SA were evaluated in the same manner as in Example 2-3. The results are illustrated in Table 4.

**[Table 4]**

| | | Amount of change in absorbance | | | | | |
|---|---|---|---|---|---|---|---|
| | | Example 2-3 | | Comparative Example 2-3 | | | |
| | | R1+POX | | R1+PEG | | R1+SA | |
| Polymer concentration of first reagent (w/v%) | Polymer concentration during reaction (w/v%) | Non-specific reactivity | CRP measurement sensitivity | Non-specific reactivity | CRP measurement sensitivity | Non-specific reactivity | CRP measurement sensitivity |
| 0 | 0.000 | 30 | 900 | 30 | 900 | 30 | 900 |
| 0.05 | 0.025 | -40 | 1260 | -30 | 2110 | 60 | 2030 |
| 0.1 | 0.050 | -40 | 1380 | 60 | 2630 | 20 | 2240 |
| 0.2 | 0.099 | 10 | 1510 | 60 | 3100 | -40 | 7030 |
| 0.25 | 0.124 | -60 | 2170 | -50 | 3560 | -110 | 8250 |
| 0.375 | 0.186 | 50 | 2530 | 50 | 5240 | 980 | 13860 |
| 0.5 | 0.248 | -90 | 3540 | 160 | 6860 | Unmeasurable^{∗} | Unmeasurable^{∗} |
| 1 | 0.495 | 20 | 5570 | 15140 | Unmeasurable^{∗} | Unmeasurable^{∗} | Unmeasurable^{∗} |
| 1.5 | 0.743 | -60 | 9660 | | | | |
| 1.75 | 0.866 | 40 | 11410 | | | | |
| 2 | 0.990 | 260 | 11670 | | | | |

The evaluation results of the non-specific reactivity and the sensitization effect by latex agglutination measurement, in a case where a R2-C solution was used as the second reagent, that is, polystyrene latex particles are used as insoluble carriers, and the polymer concentration of the first reagent is changed, are illustrated in Table 4.

Similar to the results of Example 2-2, even when the R2-C solution was used as the second reagent, that is, when polystyrene latex particles are used as the insoluble carriers, the sensitivity of R1 + POX was higher than that of R1 (Comparative Example) that does not contain a sensitizer, and the CRP measurement sensitivity was improved depending on the POX concentration. That is, it was confirmed that POX functions as a sensitizer for the latex agglutination reagent. Furthermore, R1 + POX does not have non-specific reactivity to human serum at concentrations up to 0.866%. By contrast, R1 + PEG and R1 + SA of Comparative Example 2-3 was the high sensitivity and functioned as sensitizers, while a non-specific reaction occurred. As illustrated by ^{∗} in the table, specifically, in a case where the polymer concentration of R1 + SA during the reaction is 0.248% or more and a case where the polymer concentration of R1 + PEG during the reaction is 0.495% or more, agglutination occurred rapidly when the absorbance at 0 minutes was measured, and the absorbance was 3.0 or higher (range over), which is the upper limit of measurement by the spectrophotometer, so that the measurement could not be performed (indicated in the table as unmeasurable). When comparing with the result of R1 + PEG and the result of R1 + SA of Comparative Example 2-3, assuming that the latex agglutination reagent of the present example containing poly(2-ethyl-2-oxazoline) has an amount of change in absorbance of 100 or smaller in the non-specific reactivity, the maximum sensitivity is 11,410 and can be very high. The maximum sensitivity of Comparative Examples was 5,240 for PEG and 8,250 for SA.

From the above results, in a case of using the latex agglutination reagent containing poly(2-ethyl-2-oxazoline) of the present example when polystyrene latex particles are used as the insoluble carriers, the sensitization effect could be expressed while suppressing the non-specific reaction, and the latex agglutination could be measured with high sensitivity.

### <Example 2-4: Evaluation of Measurement Accuracy>

In order to evaluate measurement accuracy, the sensitization effect (a CRP concentration of 0.5 mg/dL) by the latex agglutination measurement was repeatedly measured in the same manner as in Examples 2-2 and 2-3.

Table 5 illustrates the results of repeated measurements when the first reagent was R1 + POX and the second reagent was the R2-A solution, that is, SG particles were used as the insoluble carriers.

Table 6 illustrates the results of repeated measurements when R1 + POX was used as the first reagent and the R2-C solution was used as the second reagent, that is, polystyrene latex particles were used as the insoluble carriers.

### (Comparative Example 2-4)

For comparison, the first reagent was repeatedly measured with R1 + PEG and R1 + SA in the same manner as in Example 2-4. The results are illustrated in Table 5 and Table 6.

**[Table 5]**

| | Amount of change in absorbance in second reagent R2-A solution | | |
|---|---|---|---|
| | Example 2-4 | Comparative Example 2-4 | |
| | POX 1.5% | PEG 0.25% | SA 0.2% |
| | (CRP 0.5 mg/dL) | (CRP 0.5 mg/dL) | (CRP 0.5 mg/dL) |
| 1 | 8940 | 2770 | 8860 |
| 2 | 9290 | 2320 | 8130 |
| 3 | 9650 | 3470 | 9480 |
| 4 | 8570 | 3310 | 7030 |
| Average | 9113 | 2968 | 8375 |
| Standard deviation SD | 401 | 455 | 912 |
| Coefficient of variation CV% | 4.4 | 15.3 | 10.9 |

From the results in Table 5, it was found that R1 + POX of the present example had a smaller coefficient of variation and higher repeated measurement accuracy when SG particles were used as the insoluble carriers, as compared with Comparative Examples.

**[Table 6]**

| | Amount of change in absorbance in second reagent R2-C solution | | |
|---|---|---|---|
| | Example 1-2 | Comparative Example 1-2 | |
| | POX 1.5% | PEG 0.375% | SA 0.25% |
| | (CRP 0.5 mg/dL) | (CRP 0.5 mg/dL) | (CRP 0.5 mg/dL) |
| 1 | 9050 | 5200 | 9490 |
| 2 | 9110 | 5630 | 9290 |
| 3 | 8950 | 3140 | 7990 |
| 4 | 9660 | 5240 | 8250 |
| Average | 9193 | 4803 | 8755 |
| Standard deviation SD | 276 | 974 | 646 |
| Coefficient of variation CV% | 3 | 20.3 | 7.4 |

From the results in Table 6, it was found that R1 + POX of the present example had a smaller coefficient of variation and higher repeated measurement accuracy when polystyrene latex particles were used as the insoluble carriers, as compared with Comparative Examples.

### <Example 3: Effect of Molecular weight of Poly(2-ethyl-2-oxazoline)>

Poly(2-ethyl-2-oxazoline) (sigma-aldrich) having an average molecular weight of 200,000 was dissolved in PBS in the same manner as in Example 1-1, and a latex agglutination reagent R1 + POX-200k of the present example was prepared. Poly(2-ethyl-2-oxazoline) (sigma-aldrich) having an average molecular weight of 50,000 was dissolved in PBS in the same manner as in Example 1-1, and a latex agglutination reagent R1 + POX-50k of the present example was prepared. The non-specific reactivity and the sensitization effect by the latex agglutination measurement were evaluated in the same manner as in Example 2-2. In order to confirm the effect of the molecular weight, R1 + POX (an average molecular weight of POX 400,000) was also evaluated in the same manner. The results are illustrated in Table 7.

**[Table 7]**

| | | | | Amount of change in absorbance | |
|---|---|---|---|---|---|
| | | | | Example 3 | |
| Latex agglutination reagent | Average molecular weight of POX | Polymer concentration of first reagent (w/v%) | Polymer concentration during reaction (w/v%) | Non-specific reactivity | CRP measurement sensitivity |
| R1+POX-50k | 50,000 | 1.5 | 0.743 | 0 | 2880 |
| R1+POX-200k | 200,000 | 1.5 | 0.743 | 0 | 5090 |
| R1+POX | 400,000 | 1.5 | 0.743 | 50 | 8270 |

From the results in Table 7, it was found that in the latex agglutination reagent containing poly(2-ethyl-2-oxazoline) of the present example, the larger the molecular weight of poly(2-ethyl-2-oxazoline), the higher the sensitization effect.

### <Example 4: Evaluation 3 of Non-specific Reactivity and Sensitization Effect by Latex Agglutination Measurement>

Similar to Example 2-2, the non-specific reactivity and the sensitization effect were evaluated by the latex agglutination measurement when a R2-B solution was used as the second reagent, that is, polystyrene latex particles containing siloxane were used as insoluble carriers.

The results are illustrated in Table 8.

**[Table 8]**

| | | Amount of change in absorbance in second reagent R2-B solution | |
|---|---|---|---|
| | | Example 4 | |
| Polymer concentration of first reagent R1+POX (w/v%) | Polymer concentration during reaction (w/v%) | Non-specific reactivity | CRP measurement sensitivity |
| 0 | 0 | -20 | 220 |
| 0.5 | 0.248 | -30 | 900 |

As illustrated in Table 8, even when the R2-B solution was used as the second reagent, that is, polystyrene latex particles containing siloxane were used as the insoluble carriers, the amount of change in absorbance of R1 + POX (having a polymer concentration of the first reagent of 0.5 w/v%) was larger than that of R1 (having a polymer concentration of the first reagent of 0 w/v%) containing no sensitizer. That is, it was confirmed that POX functions as the sensitizer for the latex agglutination reagent containing polystyrene latex particles containing siloxane.

### <Example 5-1: Preparation of Latex Agglutination Reagent (First Reagent B) Containing Poly(2-ethyl-2-oxazoline)>

POX (an average molecular weight of 400,000, sigma-aldrich) was dissolved in a HEPES-T solution (pH 7.9, containing 0.01% Tween 20). The POX concentration was adjusted to be a concentration of 1.5 w/v% to obtain a latex agglutination reagent (first reagent B) of the present example. Hereinafter, this first reagent will be referred to as R1 + POX-B.

### (Comparative Example 5-1: Preparation of Latex Agglutination Reagent in Related Art)

For comparison, a POX-free first reagent (that is, HEPES-T solution) was prepared in the same manner as in Example 5-1. This is referred to as R1-B.

### <Example 6-1: Preparation Example of Second Reagent of Latex Agglutination Reagent Kit>

By a known method, commercially available polystyrene latex D particles having a particle size of 153 nm (IMMUTEX (registered trademark) P2118 manufactured by JSR Corporation) were used to prepare an antibody-sensitized latex D particle dispersion, that is, a second reagent. For antibody sensitization, an antibody was physically adsorbed on a surface of a polystyrene latex D particle and post-coated with BSA.

An example of the preparation method is illustrated below.

Polystyrene latex D particles were diluted with a HEPES buffer (pH 7.9) to have a solid content concentration of 3%. The anti-human CRP polyclonal antibodies were diluted with the same buffer to prepare an antibody solution having a concentration of about 1.0 mg/ml. 0.1 mL of the antibody solution and 0.1 mL of polystyrene latex D particles were mixed with each other and stirred at 4°C overnight. After centrifugation at 20000 G for 15 minutes, the supernatant was removed, and a 0.2% BSA HEPES solution was added to the precipitate as a blocking agent. After stirring at room temperature for 60 minutes, the supernatant was removed by centrifugation at 20000 G for 15 minutes. After washing the precipitate twice with the HEPES-T solution, the HEPES-T solution was added and redispersed so that the latex solid content concentration is finally 0.1%, and the result was used as an anti-human CRP antibody-bound latex reagent. This is called R2-D.

### <Example 6-2: Evaluation of Non-specific Reactivity and Sensitization Effect of CRP Measurement by Latex Agglutination Measurement>

Similar to Example 2-2, the non-specific reactivity and the sensitization effect were evaluated by the latex agglutination measurement when R1 + POX-B was used as the first reagent and a R2-D solution was used as the second reagent, that is, polystyrene latex particles (on which antibodies were physically adsorbed) were used as insoluble carriers. For non-specific reactivity, CRP-free human serum (a CRP concentration of 0 mg/dL) was used, and for sensitization effect, CRP solutions having CRP concentrations of 0.5, 1, 2, and 4 mg/dL were used to perform the evaluation in the same manner as in Example 2-2. The results are illustrated in Table 9.

### (Comparative Example 6)

For comparison, the non-specific reactivity and sensitization effect were evaluated in the same manner as in Example 6-2, except that the first reagent is R1-B. The results are illustrated in Table 9.

**[Table 9]**

| | Example 6 | Comparative Example 6 |
|---|---|---|
| CRP concentration (mg/dL) | Amount of change in absorbance ΔODx10000 | |
| 0 | 50 | -30 |
| 0.5 | 2500 | 670 |
| 1.0 | 6740 | 1480 |
| 2.0 | 7890 | 1740 |
| 4.0 | 8590 | 1880 |

As illustrated in Table 9, as a result of evaluating the target substance (measurement target) as CRP, the amount of change in absorbance of R1 + POX-B was larger than that of R1-B containing no sensitizer even when the R2-D solution was used as the second reagent, that is, polystyrene latex particles (on which antibodies were physically adsorbed) were used as the insoluble carriers. The non-specific reactivity was also very small. That is, it was confirmed that POX functions as the sensitizer for the latex agglutination reagent containing polystyrene latex particles (on which antibodies were physically adsorbed).

### <Example 7-1: Preparation Example of Second Reagent of Latex Agglutination Reagent Kit>

The antibody-sensitized latex D particle dispersion, that is, the second reagent was prepared in the same manner as in Example 6-1, except that anti-human ferritin antibodies (clone No. 13 manufactured by Mikuri Immunological Lab. Co.) were used as the antibodies, and the final latex solid content concentration was 0.3%. The second reagent is an anti-human ferritin antibody-bound latex reagent, which is referred to as R2-F.

### <Example 7-2: Evaluation of Non-specific Reactivity and Sensitization Effect of Ferritin Measurement by Latex Agglutination Measurement>

Similar to Example 2-2, the non-specific reactivity and the sensitization effect were evaluated by the latex agglutination measurement when R1 + POX-B was used as the first reagent and a R2-F solution was used as the second reagent, that is, polystyrene latex particles (on which antibodies were physically adsorbed) were used as insoluble carriers. The non-specific reactivity was evaluated using a phosphate-buffered saline (a ferritin concentration of 0 µg/ml), and the sensitization effect was evaluated using ferritin solutions having ferritin concentrations of 15 and 150 µg/ml as a specimen. The above described specimen was evaluated in the same manner as in Example 2-2, except that 10 µL of the specimen was used, 100 µL of the first reagent (R1 + POX-B) was used, and the optical path length of the spectrophotometer measurement cell was 2 mm. The results are illustrated in Table 10.

### (Comparative Example 7)

For comparison, the non-specific reactivity and sensitization effect were evaluated in the same manner as in Example 7-2, except that the first reagent is R1-B. The results are illustrated in Table 10.

**[Table 10]**

| | Example 7 | Comparative Example 7 |
|---|---|---|
| Ferritin concentration (µg/ml) | Amount of change in absorbance ΔOD×10000 | |
| 0 | 0 | 0 |
| 15 | 3140 | 430 |
| 150 | 7170 | 1530 |

As illustrated in Table 10, the amount of change in absorbance of R1 + POX-B was larger than that of R1-B containing no sensitizer even when the R2-F solution was used as the second reagent, that is, polystyrene latex particles (on which antibodies were physically adsorbed) were used as the insoluble carriers, and the target substance (measurement target) was ferritin. The non-specific reactivity was also very small. That is, it was confirmed that POX functions as the sensitizer for the latex agglutination reagent containing polystyrene latex particles (on which antibodies were physically adsorbed).

### <Example 8-1: Preparation Example of Second Reagent of Latex Agglutination Reagent Kit>

The antibody-sensitized latex D particle dispersion, that is, the second reagent was prepared in the same manner as in Example 6-1, except that anti-human thyroid stimulating hormone (TSH) antibodies (clone 9G11 and clone 3F10 manufactured by Mikuri Immunological Lab. Co.) were used as the antibodies, and the final latex solid content concentration was 0.3%. The second reagent is an anti-human TSH antibody-bound latex reagent, which is referred to as R2-T.

### <Example 8-2: Evaluation of Non-specific Reactivity and Sensitization Effect of TSH Measurement by Latex Agglutination Measurement>

Similar to Example 2-2, the non-specific reactivity and the sensitization effect were evaluated by the latex agglutination measurement when R1 + POX-B was used as the first reagent and a R2-T solution was used as the second reagent, that is, polystyrene latex particles (on which antibodies were physically adsorbed) were used as insoluble carriers. The non-specific reactivity was evaluated using phosphate-buffered saline containing BSA of 0.1% (a TSH concentration of 0 µg/ml), and the sensitization effect was evaluated using a TSH solution having a TSH concentration of 10 µg/ml (phosphate-buffered saline containing BSA of 0.1%) as a specimen. The above described specimen was evaluated in the same manner as in Example 2-2, except that 20 µL of the specimen was used, 100 µL of the first reagent (R1 + POX-B) was used, and the optical path length of the spectrophotometer measurement cell was 2 mm. The results are illustrated in Table 11.

### (Comparative Example 8)

For comparison, the non-specific reactivity and sensitization effect were evaluated in the same manner as in Example 8-2, except that the first reagent is R1-B. The results are illustrated in Table 11.

**[Table 11]**

| | Example 8 | Comparative Example 8 |
|---|---|---|
| TSH concentration (µg/ml) | Amount of change in absorbance ΔOD×10000 | |
| 0 | -10 | 0 |
| 10 | 440 | 60 |

As illustrated in Table 11, the amount of change in absorbance of R1 + POX-B was larger than that of R1-B containing no sensitizer even when the R2-T solution was used as the second reagent, that is, polystyrene latex particles (on which antibodies were physically adsorbed) were used as the insoluble carriers, and the target substance (measurement target) was TSH. The non-specific reactivity was also very small. That is, it was confirmed that POX functions as the sensitizer for the latex agglutination reagent containing polystyrene latex particles (on which antibodies were physically adsorbed).

While the present disclosure has been described with reference to exemplary embodiments, it is to be understood that the invention is not limited to the disclosed exemplary embodiments. The scope of the following claims is to be accorded the broadest interpretation so as to encompass all such modifications and equivalent structures and functions.

## Claims

1. A latex agglutination reaction sensitizer used in a method for measuring a target substance in a specimen by a latex agglutination method using an insoluble carrier that carries an antibody or an antigen comprising:
polyoxazoline having a repeating unit represented by the following General Formula (1), 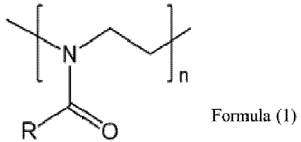
in Formula (1), R represents a hydrogen atom, a methyl group, an ethyl group, or a propyl group, and
n is an integer of 100 or greater.

2. The latex agglutination reaction sensitizer according to claim 1, wherein R in General Formula (1) is an ethyl group.

3. The latex agglutination reaction sensitizer according to claim 1 or 2, wherein the polyoxazoline has a weight average molecular weight of 10,000 or greater to 1,000,000 or smaller.

4. A latex agglutination reagent comprising:
the latex agglutination reaction sensitizer according to any one of claims 1 to 3; and
an insoluble carrier,
wherein the insoluble carrier is any one of a polystyrene particle, a polystyrene particle containing siloxane, and a polystyrene particle containing polyglycidyl (meth)acrylate.

5. The latex agglutination reagent according to claim 4, wherein a concentration of the polyoxazoline is 0.01 w/v% or more to 2.0 w/v% or less.

6. A latex agglutination reagent kit comprising the latex agglutination reagent according to claim 4 or 5 as a constituent component.

7. The latex agglutination reagent kit according to claim 6, wherein
the latex agglutination reagent contains at least two or more components, and
the latex agglutination reagent kit includes at least a first reagent including the latex agglutination reagent according to claim 4 or 5 and a second reagent containing an insoluble carrier that carries an antibody or an antigen.

8. The latex agglutination reagent kit according to claim 6 or 7, wherein the latex agglutination reagent kit is prepared to perform a latex agglutination method in which a concentration of the polyoxazoline is within a range of 0.005 w/v% or more to 1.9 w/v% or less.

9. A method for measuring a target substance in a specimen by a latex agglutination method using an insoluble carrier that carries an antibody or an antigen, wherein in the method, the latex agglutination reagent or the latex agglutination reagent kit according to any one of claims 4 to 8 is used.

10. The method for measuring a target substance in a specimen by a latex agglutination method according to claim 9, wherein the latex agglutination method in which a concentration of the polyoxazoline in the latex agglutination reagent or the latex agglutination reagent kit is within a range of 0.005 w/v% or more to 1.9 w/v% or less is performed.

11. The method for measuring a target substance in a specimen by a latex agglutination method according to claim 9 or 10, wherein the insoluble carrier is any one of a polystyrene particle, a polystyrene particle containing siloxane, and a polystyrene particle containing polyglycidyl (meth)acrylate.
